# EUROPÄISCHE PATENTSCHRIFT

(11) **EP 0 724 555 B1**
(45) Veröffentlichungstag und Bekanntmachung des Hinweises auf die Patenterteilung: **06.05.1998**
(21) Anmeldenummer: 94929532.3
(22) Anmeldetag: 12.10.1994
(51) Int. Cl.: C07C 29/80, C07C 31/125, C07C 33/02, C07C 33/025

(54) **VERFAHREN ZUR HERSTELLUNG VON FETTALKOHOLEN AUF PFLANZLICHER BASIS DURCH FRAKTIONIERUNG**
METHOD OF PRODUCING FATTY ALCOHOLS FROM VEGETABLE OILS BY FRACTIONAL DISTILLATION
PROCEDE DE PRODUCTION D'ALCOOLS GRAS A BASE VEGETALE PAR DISTILLATION FRACTIONNEE

(30) Priorität: 20.10.1993 DE 4335781
(43) Veröffentlichungstag der Anmeldung: 07.08.1996
(73) Patentinhaber: Henkel Kommanditgesellschaft auf Aktien, 40191 Düsseldorf (DE)
(72) Erfinder: KÖHLER, Michael, D-40822 Mettmann (DE); SCHMID, Karl-Heinz, D-40822 Mettmann (DE); DEMMERING, Günther, D-42653 Solingen (DE); KOMP, Horst-Dieter, D-40764 Langenfeld (DE); KUBERSKY, Hans-Peter, D-42651 Solingen (DE)
(86) Internationale Anmeldenummer: EP9403348
(87) Internationale Veröffentlichungsnummer: WO9511210

(56) Entgegenhaltungen:
- US-A- 2 004 131

## Beschreibung

Die vorliegende Erfindung betrifft ein Verfahren zu Herstellung von Fettalkoholen auf pflanzlicher Basis mit einer Iodzahl zwischen 20 und 110 und einem Konjuengehalt kleiner 4,5 Gew.-%, Fettalkohole auf pflanzlicher Basis mit einer Iodzahl von 20 bis 110 und einem Konjuengehalt kleiner 4,5 Gew.-% sowie die Verwendung dieser Produkte zur Herstellung obenflächenaktiver Mittel.

### Stand der Technik

Fettstoffe, insbesondere ungesättigte Fettalkohole, sind wichtige Zwischenprodukte für eine große Anzahl von Erzeugnissen der chemischen Industrie, wie z.B. für die Herstellung von Tensiden und die Herstellung von Hautpflegeprodukten. Eine Übersicht hierzu findet sich beispielsweise von U. Ploog et al. in **Seifen-Öle-Fette-Wachse 109, 225 (1983)**. Zu ihrer Herstellung geht man von mehr oder minder ungesättigten Fettsäuremethylestern aus, die beispielsweise in Gegenwart chrom- oder zinkhaltiger Mischkatalysatoren hydriert werden "können **[Ullmam's Enzyclopaedie der technischen Chemie, Verlag Chemie, Weinheim, 4. Aufl., Bd. 11, S. 436f].**

Stand der Technik ist ein großtechnisches Verfahren, wie es bislang auch bei der Anmelderin durchgeführt worden ist, nach dem tierische Fette und Öle eingesetzt werden, die nach der Hydrierung anfallenden ungesättigten Fettalkohole bei einer Sumpftemperatur von z. B. 220 bis 250°C und einem verminderten Druck von 1 bis 20 mbar - gemessen am Kolonnenkopf - destilliert werden. Da die Herstellung von ungesättigten Fettalkoholen mit hohen Kosten verbunden ist, wurde auf einen möglichst geringen Rohstoffverlust destilliert. Tatsächlich konnte auf diese Weise eine Ausbeute von ca. 90 % der Theorie - und entsprechend ein Verlust von 10 % - erreicht werden, die Produkte zeigten jedoch einen deutlichen Eigengeruch. Ein weiterer Nachteil besteht ferner darin, daß die Fettalkohole des Standes der Technik ein unbefriedigendes Lager- und Kälteverhalten zeigen.

Aus anwendungstechnischen Gründen sind ungesättigte Fettalkohole mit Iodzahlen von 20 bis 95 besonders bevorzugt, da diese einen für die Verwendung in Kosmetikprodukten besonders günstigen Erstarrungspunkt besitzen. Ungesättigte Fettalkohole mit Iodzahlen im o.g. Bereich sind bis jetzt nur aus Fetten auf tierischer Basis bekannt. Der gewünschte Iodzahlbereich wird durch Verschneiden verschiedener Produkte mit unterschiedlichen Iodzahlbereichen eingestellt. Die Einstellung des Iodzahlbereichs durch destillative Verfahren ist nicht möglich, da die Iodzahl bzw. der Iodzahlbereich von Fettalkoholen bzw. Fettsäuren auf tierischer Basis bei der Fraktionierung nahezu konstant bleibt.

Tierische Fette haben aber den Nachteil, daß sie sehr heterogen aufgebaut sind. Zum Beispiel enthalten tierische Fette stickstoffhaltige Verbindungen wie Amide oder Steroide, wie z. B. Cholesterin, die direkt oder indirekt für den oben erwähnten unangenehmen Geruch der Produkte verantwortlich sind. Die stickstoffhaltigen Verbindungen können Nebenreaktionen eingehen, was die Produktstabilität, insbesondere die Oxidationsstabilität, verschlechtert und zu verfärbten Produkten führt.

Gerade auf dem Kosmetikmarkt besteht ein dringendes Bedürfnis nach immer reineren und qualitativ hochwertigeren Rohstoffen, eine Forderung, die üblicherweise nur durch immer aufwendigere technische Verfahren und zusätzliche Reinigungsschritte zur Verfügung gestellt werden können. Im Fall der ungesättigten Fettalkohole besteht dabei insbesondere das Bedürfnis nach Produkten mit verbesserter Farb- und Geruchsqualität sowie vorteilhafterem Kälteverhalten. Hinzukommt, daß sich in den letzten Jahren das Verbraucherverhalten dahingehend verändert hat, daß die Verbraucher sehr großen Wert auf rein pflanzliche Produkte legen.

Die bekannten pflanzlichen Fettalkohole weisen Iodzahlen in dem Bereich unter 20 oder sehr hohe Iodzahlen über 100 auf. Fettalkohole mit Iodzahlen in dem oben genannten anwendungstechnisch besonders bevorzugten Bereich zwischen 20 und 95 sind nicht bekannt. Das Verschneiden der Fettalkohole mit sehr unterschiedlichen Iodzahlen führt nicht zu zufriedenstellenden Produkten.

Die Aufgabe der vorliegenden Erfindung ist es Fettalkohole, auf pflanzlicher Basis zur Verfügung zu stellen, welche Iodzahlen in dem anwendungstechnisch besonders bevorzugten Bereich aufweisen und gleichzeitig gegenüber den ungesättigten Fettalkoholen auf tierischer Basis eine größere Oxidationsstabilität und ein gleichwertiges oder besseres Kälteverhalten aufweisen.

### Beschreibung der Erfindung

Gegenstand der vorliegenden Erfindung ist ein Verfahren zur Herstellung von Fettalkoholen auf pflanzlicher Basis mit einer Iodzahl zwischen 20 und 110 und einem Konjuengehalt kleiner 4,5 Gew.-%, die im wesentlichen ungesättigte Fettalkohole und Gemische aus gesättigten und ungesättigten Fettalkoholen mit der allgemeinen Formel (I) enthalten,

**R**^{**1**}**OH (I)**

worin R¹ für einen gesättigten oder ungesättigten, linearen oder verzweigten Alkylrest mit 8 bis 22 Kohlenstoffatomen steht, bei dem man pflanzliche Telle und Öle a) durch Druckspaltung in die Fettsäuren bzw. durch nachfolgende veresterung oder direkte umesterung mit Methanol in die Fettsäuremethylester überfürhrt und b) die Fettsäuren bzw. Fettsäuremethylester unter Erhalt der Doppelbindungen in den entsprechenden Fettalkoholen hydriert, mit der Maßgabe, daß man die Fettsäuren, die Fettsäuremethylester und/oder die Hydrierprodukte einer Fraktionierung unterwirft, in der, ein Vorlauf in einer solchen Menge abgenommen wird, daß das Endprodukt eine Iodzahl von 20 bis 110 aufweist.

Überraschenderweise wurde gefunden, daß es im Gegensatz zu den Produkten auf tierischer Basis möglich ist, Fettalkohole auf pflanzlicher Basis in dem oben genannten Iodzahlbereich herzustellen, die im wesentlichen ungesättigte Fettalkohole und Gemische aus gesättigten und ungesättigten Fettalkoholen mit der allgemeinen Formel (I) enthalten, wobei die Iodzahl entsprechend den anwendungstechnischen Anforderungen durch einfache Fraktionierung auf einen gewünschten Bereich eingestellt werden kann. Die nach dem erfindungsgemäßen Verfahren erhaltenen Produkte auf pflanzlicher Basis weisen eine bessere Oxidationsstabilität und einen geringeren Eigengeruch auf als die entsprechenden Produkte auf tierischer Basis.

Ein weiterer Gegenstand der vorliegenden Erfindung sind Fettalkohole auf pflanzlicher Basis mit einer Iodzahl, im Bereich von 20 bis 110 und einem Konjuengehalt kleiner 4,5 Gew.-% die im wesentlichen ungesättigte Fettalkohole und Gemische aus gesättigten und ungesättigten Fettalkoholen mit der allgemeinen Formel (I) enthalten,

**R**^{**1**}**OH (I)**

worin R¹ für einen gesättigten oder ungesättigten, linearen oder verzweigten Alkylrest mit 8 bis 22 Kohlenstoffatomen steht ,dadurch erhältlich, daß man pflanzliche Fette und Öle
a) durch Druckspaltung in die Fettsäuren bzw. durch nachfolgende Veresterung oder direkte Umesterung mit Methanol in die Fettsäuremethylester überführt und
b) die Fettsäuren bzw. Fettsäuremethylester unter Erhalt der Doppelbindungen zu den entsprechenden Fettalkohole hydriert,
mit der Maßgabe, daß man die Fettsäuren, die Fettsäuremethylester und/oder die Hydrierprodukte einer Fraktionierung unterwirft, in der ein Vorlauf in einer solchen Menge abgenommen wird, daß das Endprodukt eine Iodzahl im Bereich von 20 bis 110 aufweist. Bevorzugt weisen die erfindungsgemäßen Fettalkohole auf pflanzlicher Basis eine Iodzahl von 40 bis 85, auf. Außerdem sind Verbindungen mit der Formel (I) bevorzugt, worin R¹ für einen Alkylrest mit 12 bis 20 Kohlenstoffatomen steht.

Die erfindungsgemäßen Fettalkohole weisen aufgrund des geringen Konjuengehalts eine besonders hohe Stabilität auf.

Zur Durchführung des erfindungsgemäßen Verfahrens geht man von ungesättigten bzw. teilweise ungesättigten pflanzlichen Fetten und Ölen aus. Besonders bevorzugt sind hierbei Palmöl, Palmstearinöl, Palmkernoleinöl, Kokosöl, Palmkernöl, Sonnenblumenöl, neuem Rüböl, Sojaöl, Erdnußöl, Rapsöl, Leinöl und Olivenöl. Die im wesentlichen aus Triglyceriden bestehenden Fettstoffe werden in an sich bekannter Weise durch Druckspaltung in die Fettsäuren überführt und ggf. anschließend mit Methanol verestert oder mit Methanol in den Fettsäuremethylester umgeestert. Die Fettsäure bzw. der Fettsäuremethylester werden anschließend nach im Stand der Technik bekannten Verfahren zu den korrespondierenden Fettalkoholen hydriert. Der Anteil der enthaltenen gesättigten und ungesättigten Bestandteile sowie die Kettenlängenverteilung ergibt sich aus den eingesetzten pflanzlichen Ölen. In den obigen Verbindungen mit der Formel (I) steht R¹ für einen Alkylrest mit 8 bis 22 Kohlenstoffatomen, bevorzugt 12 bis 20 Kohlenstoffatomen.

Durch den Einsatz der pflanzlichen Fette und Öle als Ausgangsprodukte stellen die eingesetzten Fettsäuren, Fettsäuremethylester bzw. das Hydrierprodukt Gemische aus Fettsäuren, Fettsäuremethylestern bzw. Fettalkoholen unterschiedlicher Kettenlänge dar. Erfindungsgemäß wird die Iodzahl der herzustellenden Fettalkohole eingestellt, indem man die aus der Druckspaltung erhaltenen Fettsäuren, die aus der Umesterung der Triglyderide erhaltenen Fettsäuremethylester oder das Hydrierprodukt, das aus der Hydrierung der Fettsäure oder des Fettsäuremethylester erhalten wird, fraktioniert. Vor der Fraktionierung wird die Iodzahl des zu fraktionierenden Produktes bestimmt. In Abhängigkeit vom Ausgangsprodukt bzw. dessen Iodzahl und von der gewünschten Iodzahl wird bei der Fraktionierung eine bestimmte Vorlaufmenge entnommen. Durch die Entnahme des Vorlaufs wird die Iodzahl des Fettalkohols erhöht. Zur Einstellung der Iodzahl des Produktes wird bevorzugt während der Fraktionierung die Iodzahl des noch nicht überdestillierten Produktes kontrolliert. Nach dem erfindungsgemäßen Verfahren ist es z.B. möglich, aus Kokos/Palmkernöl eine Fettsäure- oder FettsäuremethylesterFraktion zu erhalten, die als Hauptbestandteile Fettsäure bzw. Fettsäuremethylester mit Kettenlängen von 16 bis 18 Kohlenstoffatomen enthält, sog. C_{16/18}-Fettsäure bzw. -Fettsäuremethylester. Die gewünschte Kettenlängenverteilung kann auch durch entsprechende Fraktionierung des Fettalkohols eingestellt werden.

Die Fraktionierbedingungen der beispielsweise aus der Hydrierung erhaltenen ungesättigten Fettalkohole unter vermindertem Druck sind seit langem bekannt. Die Fraktionierung kann batchweise oder kontinuierlich bei verringertem Druck durchgeführt werden. Die Beheizung kann beispielsweise durch Heißdampf erfolgen, wobei eine Sumpftemperatur von z. B. 220 bis 250°C vorliegt.

Die eigentliche Fraktionierung findet in einer gepackten Kolonne mit druckverlustarmen Einbauten statt. Als Einbauten kommen beispielsweise geordnete Blechpackungen in Betracht. weitere Beispiele finden sich in **RÖMPP Chemie Lexikon, Thieme Verlag, Stuttgart, 9. Auflage, Bd. 3, S. 2305 (1990)** unter dem Stichwort "Kolonnen-Einbauten" und in der dort genannten Literatur.

Das erforderliche Feinvakuum von 1 bis 20 mbar am Kopf der Kolonne kann beispielsweise mit Hilfe von Wasserringpumpen und vorgeschalteten Dampf strahlern erzielt werden. Vorzugsweise sollte der Druckabfall über die gesamte Destillationsanlage nicht mehr als 20 mbar betragen.

Eine Produktverbesserung kann erhalten werden, wenn die ungesättigten Fettalkohole so destilliert werden, daß ein Rückstand bis zu 10 Gew.-%, bevorzugt 2 bis 7 Gew.-%, anfällt. Durch diese Verfahrensmaßnahme werden die Farbzahl und der Geruch der Endprodukte weiter deutlich verbessert.

### Gewerbliche Anwendbarkeit

Die nach dem erfindungsgemäßen Verfahren erhältlichen ungesättigten Fettalkohole auf pflanzlicher Basis sind farb- und geruchsarm und weisen ein besonders vorteilhaftes Kälteverhalten auf. Sie eignen sich daher als Rohstoffe zur Herstellung von Wasch-, Spül- und Reinigungsprodukten sowie Produkten zur Haar- und Körperpflege, in denen sie in Mengen von 1 bis 50, vorzugsweise 5 bis 30 Gew.-% - bezogen auf die Mittel - enthalten sein können.

Die Erfindung wird anhand der folgenden Beispiele näher erläutert:

### Beispiele

In einer üblichen Hydrieranlage wurden Fettsäuremethylester bei einem Druck von ca. 225 bar und einer Temperatur von 275 bis 330°C in Gegenwart eines CuCrO₄-Katalysators hydriert. Das Produkt aus der Hydrierung wird destilliert, wobei ein entsprechender Vorlauf entnommen wird.

### Beispiel 1

Ein C_{12/18}-Palmkernmethylester, der aus der Umesterung von Palmkernöl und anschließender Fraktionierung erhalten wurde, wurde zu einem C_{12/14}- und C_{16/18}-Methylester fraktioniert. Der C_{16/18}-Methylester wurde in der oben beschriebenen Weise zum Fettalkohol hydriert. Dieses Rohprodukt wurde in einer zweistufigen Vakuum-Praktionieranlage destilliert, wobei ein Vorlauf von 3 Gew.-% entnommen wurde und ein Rückstand von 3 Gew.-% verblieb.

Es wurde ein Fettalkohol (FA) mit der folgenden Kettenverteilung und den folgenden Kennzahlen erhalten:

| | |
|---|---|
| FA C 12 | 0,2 Gew.-% |
| FA C 14 | 4,5 Gew.-% |
| FA C 15 | 0,3 Gew.-% |
| FA C 16 | 25,1 Gew.-% |
| FA C 16' | 0,4 Gew.-% |
| FA C 17 | 0,3 Gew.-% |
| FA C 18 | 8,9 Gew.-% |
| FA C 18' | 52,5 Gew.-% |
| FA C 18" | 2,9 Gew.-% |
| FA C 18" Konj. | 3,2 Gew.-% |
| FA C 18"' | 0,1 Gew.-% |
| FA C 20 | 0,6 Gew.-% |
| | |
| Säurezahl | = 0,02 |
| Verseifungszahl | = 0,35 |
| OH-Zahl | = 213,5 |
| Iodzahl | = 61,3 |
| H₂O-Gehalt | = 0,02 |
| Erweichungspunkt | = 26,6°C |
| Kohlenwasserstoffgehalt | = 0,87 Gew.-% |
| CO-Zahl | = 360 |
| Hazen | = < 10 |

### Beispiel 2

Das in Beispiel 1 aus der Hydrierung erhaltene Produkt wird derart destilliert, daß eine Vorlauf-Fraktion von etwa 18 Gew.-% entnommen wird, wobei ein Fettalkohol mit einer höheren Iodzahl von 75 erhalten wird.

Dieser Fettalkohol weist die folgende Kettenverteilung und die folgenden Kennzahlen auf:

| | |
|---|---|
| FA C 12 | 0,0 Gew.-% |
| FA C 14 | 0,1 Gew.-% |
| FA C 15 | 0,1 Gew.-% |
| FA C 16 | 12,1 Gew.-% |
| FA C 16' | 0,2 Gew.-% |
| FA C 17 | 0,3 Gew.-% |
| FA C 18 | 11,5 Gew.-% |
| FA C 18' | 66,5 Gew.-% |
| FA C 18'' | 3,7 Gew.-% |
| FA C 18" Konj. | 4,3 Gew.-% |
| FA C 18''' | 0,2 Gew.-% |
| FA C 20 | 0,4 Gew.-% |
| | |
| Säurezahl | = 0,02 |
| Verseifungszahl | = 0,40 |
| OH-Zahl | = 210,5 |
| Iodzahl | = 75 |
| H₂O-Gehalt | = 0,02 |
| Erweichungspunkt | = 23,2°C |
| Kohlenwasserstoffgehalt | = 0,13 Gew.-% |
| CO-Zahl | = 306 |
| Hazen | = 5 |

### Beispiel 3

Ausgehend von einer Mischung aus einem C_{16/18}-Palmkernmethylester (70 Gew.-%) und Rübölmethylester (30 Gew.-%), hergestellt aus neuem Rüböl, wurde entsprechend Beispiel 1 ein Fettalkohol mit der folgenden Kettenverteilung und den folgenden Kennzahlen hergestellt:

| | |
|---|---|
| FA C 12 | 0,0 Gew.-% |
| FA C 14 | 0,0 Gew.-% |
| FA C 15 | 0,2 Gew.-% |
| FA C 16 | 23,1 Gew.-% |
| FA C 16' | 0,8 Gew.-% |
| FA C 17 | 0,4 Gew.-% |
| FA C 18 | 6,4 Gew.-% |
| FA C 18' | 60,3 Gew.-% |
| FA C 18" | 4,6 Gew.-% |
| FA C 18" Konj. | 3,2 Gew.-% |
| FA C 18"' | 0,1 Gew.-% |
| FA C 20 | 0,6 Gew.-% |
| Säurezahl | = 0,02 |
| Verseifungszahl = | 0,35 |
| OH-Zahl | = 213 |
| Iodzahl | = 73,9 |
| H₂O-Gehalt | = 0,03 |
| Erweichunspunkt | = 22,9°C |
| Kohlenwasserstoffgehalt = | 0,2 Gew.-% |
| CO-Zahl | = 279 |
| Hazen | = < 10 |

## Patentansprüche

1. Fettalkohole auf pflanzlicher Basis mit einer Iodzahl im Bereich von 20 und 1 10 und einem Konjuengehalt kleiner 4,5 Gew.-%, die im wesentlichen ungesättigte Fettalkohole und Gemische aus gesättigten Fettalkoholen der Formel **(I)** enthalten,
**R**^{**1**}**OH (I)**
in der R¹ für einen gesättigten oder ungesättigten, linearen oder verzweigten Alkylrest mit 8 bis 22 Kohlenstoffatomen steht, dadurch erhältlich, daß man pflanzliche Fette und Öle
a) durch Druckspaltung in die Fettsäuren bzw. durch nachfolgende Veresterung oder direkte Umesterung mit Methanol in die Fettsäuremethylester überführt und
b) die Fensäuren bzw. Fettsäuremethylester unter Erhalt der Doppelbindungen zu den entsprechenden Fettalkohole hydriert,
mit der Maßgabe, daß man die Fettsäuren, die Fettsäuremethylester und/oder die Hydrierprodukte einer Fraktionierung unterwirft, in der ein Vorlauf in einer solchen Menge abgenommen wird, daß das Endprodukt eine Iodzahl im Bereich von 20 bis 110 aufweist.

2. Verfahren zur Herstellung von Fettalkoholen auf pflanzlicher Basis mit einer Iodzahl im Bereich von 20 und 110 und einem Konjuengehalt kleiner 4,5 Gew.-%, die im wesentlichen ungesättigte Fettalkohole und Gemische aus gesättigten Fettalkoholen der Formel **(I)** enthalten,
**R**^{**1**}**OH (I)**
in der R¹ für einen gesättigten oder ungesättigten, linearen oder verzweigten Alkylrest mit 8 bis 22 Kohlenstoffatomen steht, bei dem man pflanzliche Fette und Öle
a) durch Druckspaltung in die Fettsäuren bzw. durch nachfolgende Veresterung oder direkte Umesterung mit Methanol in die Fettsäuremethylester überführt und
b) die Fensäuren bzw. Fettsäuremethylester unter Erhalt der Doppelbindungen zu den entsprechenden Fettalkoholen hydriert,
mit der Maßgabe, daß man die Fettsäuren, die Fettsäuremethylester und/oder die Hydrierprodukte einer Fraktionierung unterwirft, in der ein Vorlauf in einer solchen Menge abgenommen wird, daß das Endprodukt eine Iodzahl im Bereich von 20 bis 110 aufweist.

3. Verwendung der Fettalkohole nach Anspruch 1 zur Herstellung von Wasch-, Spül- und Reinigungsmitteln sowie Mitteln zur Haar und Körperpflege.

## Claims

1. Fatty alcohols based on vegetable fats and oils with an iodine value in the range from 20 to 110 and a conjugene content of less than 4.5% by weight which contain substantially unsaturated fatty alcohols and mixtures of saturated fatty alcohols corresponding to formula **(I)**:
**R**^{**1**}**OH (I)**
where R¹ is a saturated or unsaturated, linear or branched alkyl group containing 8 to 22 carbon atoms,
obtainable by
a) converting vegetable fats and oils into the fatty acids by pressure hydrolysis or into the fatty acid methyl esters by subsequent esterification or direct transesterification with methanol and
b) hydrogenating the fatty acids or fatty acid methyl esters to the corresponding fatty alcohols with the double bonds intact,
with the proviso that the fatty acids, the fatty acid methyl esters and/or the hydrogenation products are fractionated by removing a head fraction in such a quantity that the end product has an iodine value of 20 to 110.

2. A process for the production of fatty alcohols based on vegetable fats and oils with an iodine value in the range from 20 to 110 and a conjugene content of less than 4.5% by weight which contain substantially unsaturated fatty alcohols and mixtures of saturated fatty alcohols corresponding to formula **(I)**:
**R**^{**1**}**OH (I)**
where R¹ is a saturated or unsaturated, linear or branched alkyl group containing 8 to 22 carbon atoms,
in which vegetable fats and oils
a) are converted into the fatty acids by pressure hydrolysis or into the fatty acid methyl esters by subsequent esterification or direct transesterification with methanol and
b) the fatty acid or fatty acid methyl ester is hydrogenated to the corresponding fatty alcohol with the double bonds intact,
with the proviso that the fatty acids, the fatty acid methyl esters and/or the hydrogenation products are fractionated by removing a head fraction in such a quantity that the end product has an iodine value of 20 to 110.

3. The use of the fatty alcohols claimed in claim 1 for the production of laundry detergents, dishwashing detergents and cleaning products and hair-care and body-care products.

## Revendications

1. Alcools gras à base végétale ayant un indice d'iode compris entre 20 et 110 et une teneur en matière de conjugaison inférieure à 4,5 % en poids, qui contiennent des alcools gras essentiellement insaturés et des mélanges d'alcools gras saturés de formule (I)
R¹OH (I)
dans laquelle R¹ représente un radical alkyle saturé ou insaturé, linéaire ou ramifié, ayant de 8 à 22 atomes de carbone, que l'on peut obtenir en faisant subir le traitement suivant à des graisses et à des huiles végétales
a) par coupure sous pression dans les acides gras ou selon les cas par estérification ultérieure ou transestérification directe avec du méthanol, on effectue une transformation pour donner les esters méthyliques d'acide gras et
b) on hydrogène les acides gras ou selon les cas les esters méthyliques d'acide gras en maintenant les doubles liaisons pour donner les alcools gras correspondants,
sous réserve qu'on soumet les acides gras, les esters méthyliques d'acides gras et/ou les produits d'hydrogénation à un fractionnement dans lequel on retire des têtes de distillation en une quantité telle que le produit final présente un indice d'iode compris entre 20 à 110.

2. Procédé de fabrication d'alcools gras à base végétale ayant un indice d'iode comprise entre 20 et 110 et une teneur en matière de conjugaison inférieure à 4,5 % en poids, qui contiennent des alcools gras essentiellement insaturés et des mélanges d'alcools gras saturés de formule (I)
R¹OH (I)
dans laquelle R¹ représente un radical alkyle saturé ou insaturé, linéaire ou ramifié ayant de 8 à 22 atomes de carbone, dans lequel on soumet aux opérations suivantes des matières grasses et des huiles végétales :
a) par coupure sous pression dans les acides gras ou selon les cas par estérification ultérieure ou transestérification directe avec du méthanol, on effectue une transformation avec du méthanol pour obtenir les esters en méthyliques d'acide gras, et
b) on hydrogène les acides gras ou selon les cas les esters méthyliques d'acide gras avec maintien des doubles liaisons pour donner les alcools gras correspondants,
sous réserve qu'on soumet les acides gras, les esters méthyliques d'acide gras et/ou les produits d'hydrogénation à un fractionnement dans lequel on prélève des têtes de distillation en une quantité telle que le produit final présente un indice d'iode compris entre 20 et 110.

3. Utilisation des alcools gras selon la revendication 1 pour la fabrication d'agents de lavage, de nettoyage, de purification, ainsi que d'agents pcur le soin des cheveux et du corps.
